# EUROPEAN PATENT APPLICATION

(11) **EP 2 050 407 A1**
(43) Date of publication of application: **22.04.2009**
(21) Application number: 09151457.0
(22) Date of filing: 29.12.2004
(51) Int. Cl.: A61B 17/56, A61B 17/70

(54) **Bone anchor assemblies**

(30) Priority: 30.12.2003 US 533408 P
(62) Divisional of application: 04817077.3
(71) Applicant: DePuy Spine Sàrl, 2400 Le Locle (CH)
(72) Inventor: Doherty, Thomas, V., Latham, NY 12110 (US); Hall, Mark, T., Bridgewater, MA 02324 (US); Runco, Thomas, Canton, MA 02021 (US)
(74) Representative: Orr, Robert

(57) **Abstract**

A bone anchor assembly may include a bone anchor having a proximal head and a distal shaft configured to engage bone and a receiving member for receiving a spinal fixation element to be coupled to the bone anchor. The receiving member may have a first end having a first bore defining a first bore axis, a recess in communication with the first bore, and a second end having a second bore sized to receive at least a portion of the bone anchor. The second bore may define a second bore axis that intersects the first bore axis and may have a first opening through which the at least a portion of the bone anchor extends and a second opening opposite the first opening. The second opening may be sized to pass the head of the bone anchor during assembly of the bone anchor assembly.

## Description

### Background

Spinal fixation systems may be used in orthopedic surgery to align and/or fix a desired relationship between adjacent vertebrae. Such systems typically include a spinal fixation element, such as a relatively rigid fixation rod or plate, that is coupled to adjacent vertebrae by attaching the element to various anchoring devices, such as hooks, bolts, wires, or screws. The spinal fixation element can have a predetermined contour that has been designed according to the properties of the target implantation site, and once installed, the spinal fixation element holds the vertebrae in a desired spatial relationship, either until desired healing or spinal fusion has taken place, or for some longer period of time.

Spinal fixation elements can be anchored to specific portions of the vertebra. Since each vertebra varies in shape and size, a variety of anchoring devices have been developed to facilitate engagement of a particular portion of the bone. Pedicle screw assemblies, for example, have a shape and size that is configured to engage pedicle bone. Such screws typically include a threaded shank that is adapted to be threaded into a vertebra, and a head portion having a spinal fixation element receiving element, which, in spinal rod applications, is usually in the form of a U-shaped slot formed in the head for receiving the rod. A set-screw, plug, cap or similar type of closure mechanism, may be used to lock the rod into the rod-receiving portion of the pedicle screw. In use, the shank portion of each screw may be threaded into a vertebra, and once properly positioned, a fixation rod may be seated through the rod-receiving portion of each screw and the rod is locked in place by tightening a cap or similar type of closure mechanism to securely interconnect each screw and the fixation rod. Other anchoring devices also include hooks and other types of bone screws.

In certain procedures, it may be difficult to position bone anchors on adjacent vertebrae because the close proximity of the adjacent vertebrae can result in interference between the bone anchors. In cervical vertebrae, for example, it is frequently necessary to pivot the bone anchors out of alignment with one another to avoid such interference.

### Summary

Disclosed herein are bone anchor assemblies and methods of engaging a bone anchor assembly to bone that facilitate engagement of the bone anchor assembly to a bone, such as a vertebra. Also disclosed herein are methods of manufacturing a bone anchor assembly.

In one exemplary embodiment, a bone anchor assembly may comprise a bone anchor having a proximal head and a distal shaft configured to engage bone and a receiving member for receiving a spinal fixation element to be coupled to the bone anchor. In the exemplary embodiment, the receiving member may have a first end having a first bore defining a first bore axis, a recess in communication with the first bore, and a second end having a second bore sized to receive at least a portion of the bone anchor. The second bore may define a second bore axis that intersects the first bore axis and may have a first opening through which the at least a portion of the bone anchor extends and a second opening opposite the first opening. The second opening may be sized to pass the head of the bone anchor during assembly of the bone anchor assembly.

An exemplary method of engaging a bone anchor assembly to a bone of a patient may comprise delivering a bone anchor assembly to proximate the bone. The bone anchor assembly may comprise a bone anchor having a proximal head and a distal shaft configured to engage bone and a receiving member. The receiving member may have a first end having a first bore defining a first bore axis, a recess in communication with the first bore, and a second end having a second bore sized to receive at least a portion of the bone anchor. The second bore, in the exemplary embodiment, may define a second bore axis that intersects the first bore axis. The second bore may having a first opening through which the at least a portion of the bone anchor extends and a second opening opposite the first opening. The exemplary method may comprise inserting a tool through the second opening in the second bore to engage the bone anchor.

An exemplary method of manufacturing a bone anchor assembly may comprise providing a receiving member having a first end having a first bore defining a first bore axis, a recess in communication with the first bore, and a second end having a second bore that defines a second bore axis. In the exemplary embodiment, the second bore axis may intersect the first bore axis. The second bore, in the exemplary embodiment, may have a first opening and a second opening opposite the first opening. The exemplary method may comprise positioning a bone anchor through the second opening in the second bore.

### Brief Description of the Figures

These and other features and advantages of the bone anchor assemblies and methods disclosed herein will be more fully understood by reference to the following detailed description in conjunction with the attached drawings in which like reference numerals refer to like elements through the different views. The drawings illustrate principles of the instruments disclosed herein and, although not to scale, show relative dimensions.
FIGURE 1 is a perspective view of an exemplary embodiment of a bone anchor assembly illustrating a spinal rod coupled to the bone anchor assembly;
FIGURE 2 is a top view of the bone anchor assembly of FIGURE 1;
FIGURE 3 is a side elevational view in cross-section of the bone anchor assembly of FIGURE 1 taken along line C-C of FIGURE 2;
FIGURE 4 is an exploded assembly view of the components of the bone anchor assembly of FIGURE 1;
FIGURE 5 is side elevational view in cross section of the components of the bone anchor assembly of FIGURE 1;
FIGURES 6A-6B are perspective views of the receiving member of the bone anchor assembly of FIGURE 1;
FIGURE 7 is a top view of the receiving member of the bone anchor assembly of FIGURE 1;
FIGURE 8 is a side elevational view of the receiving member of the bone anchor assembly of FIGURE 1;
FIGURE 9 is a front view of the receiving member of the bone anchor assembly of FIGURE 1;
FIGURE 10 is a side elevational view in cross section of the receiving member of the bone anchor assembly of FIGURE 10 taken along the line B-B of FIGURE 9;
FIGURES 11A-11B are perspective views of the compression member of the bone anchor assembly of FIGURE 1;
FIGURE 12 is a top view of the compression member of the bone anchor assembly of FIGURE 1;
FIGURE 13 is an exploded assembly view of the components of an exemplary embodiment of a bone anchor assembly;
FIGURE 14 is side elevational view in cross section of the components of the bone anchor assembly of FIGURE 13;
FIGURE 15 is a side view of an exemplary embodiment of a bone anchor assembly having a receiving member with a reduced diameter first end;
FIGURE 16 is a side elevational view in cross section of the bone anchor assembly of FIGURE 15;
FIGURE 17 is a side elevational view of an exemplary embodiment of a bone anchor assembly illustrating the range of angular variation of the bone anchor;
FIGURE 18 is a rear perspective view of the bone anchor assembly of FIGURE 17;
FIGURE 19 is a perspective view of the receiving member of the bone anchor assembly of FIGURE 17;
FIGURE 20 is a bottom view of the receiving member of the bone anchor assembly of FIGURE 17, illustrating the opening of the second bore of the receiving member;
FIGURE 21 is a side elevational view in cross section of the receiving member of the bone anchor assembly of FIGURE 17 taken along the line H-H of FIGURE 20;
FIGURE 22 is a side elevational view of an exemplary embodiment of a bone anchor assembly;
FIGURE 23 is a top view of the receiving member of the bone anchor assembly of FIGURE 22, illustrating the bore axis of the second bore of the receiving member offset from the bore axis of the first bore of the receiving member;
FIGURE 24 is a side elevational view in cross section of the bone anchor assembly of FIGURE 22; and
FIGURE 25 is a side elevational view of an exemplary embodiment of a bone anchor assembly, illustrating a rod positioned in the angled recess of the receiving member.

### Detailed Description

Certain exemplary embodiments will now be described to provide an overall understanding of the principles of the structure, function, manufacture, and use of the bone anchor assemblies disclosed herein. One or more examples of these embodiments are illustrated in the accompanying drawings. Those of ordinary skill in the art will understand that the bone anchor assemblies specifically described herein and illustrated in the accompanying drawings are non-limiting exemplary embodiments and that the scope of the present invention is defined solely be the claims. The features illustrated or described in connection with one exemplary embodiment may be combined with the features of other embodiments. Such modifications and variations are intended to be included within the scope of the present invention.

The articles "a" and "an" are used herein to refer to one or to more than one (i.e. to at least one) of the grammatical object of the article. By way of example, "an element" means one element or more than one element.

The term "distal" as used herein with respect to any component or structure will generally refer to a position or orientation that is proximate, relatively, to the bone surface to which a bone anchor is to be applied. Conversely, the term "proximal" as used herein with respect to any component or structure will generally refer to a position or orientation that is distant, relatively, to the bone surface to which a bone anchor is to be applied.

The terms "comprise," "include," and "have," and the derivatives thereof, are used herein interchangeably as comprehensive, open-ended terms. For example, use of "comprising," "including," or "having" means that whatever element is comprised, had, or included, is not the only element encompassed by the subject of the clause that contains the verb.

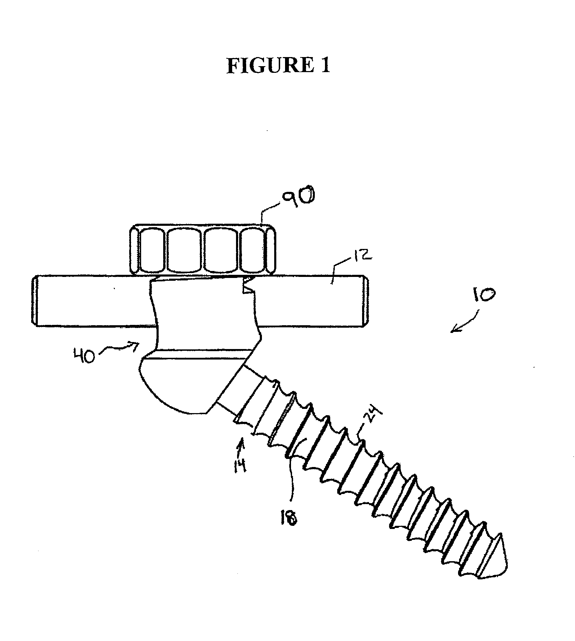

FIGURES 1-5 illustrate an exemplary embodiment of a bone anchor assembly 10 coupled to an exemplary spinal fixation element, a spinal rod 12. The exemplary bone anchor assembly 10 may be employed to engage one or more spinal fixation elements to bone. For example, bone anchor assembly 10 may be employed to fix a spinal plate, rod, and/or cable to a vertebra of the spine. Although the exemplary bone anchor assembly 10 described below is designed primarily for use in spinal applications, one skilled in the art will appreciate that the structure, features, and principles of the exemplary bone anchor assembly 10, as well as the other exemplary embodiments described below, may be employed to couple any type of orthopedic implant to any type of bone or tissue. Non-limiting examples of applications of the bone fixation anchor assembly 10 described herein include long bone fracture fixation/stabilization, small bone stabilization, lumbar spine as well as thoracic stabilization/fusion, cervical spine compression/fixation, and skull fracture/reconstruction plating.

The illustrated exemplary bone anchor 10 may include a bone anchor 14 having a proximal head 16 and a distal shaft 18 configured to engage bone. The distal shaft 18 of the bone anchor 14 has a shaft diameter 20 and a longitudinal axis 22. The distal shaft 18 may include one or more bone engagement mechanisms to facilitate gripping engagement of the bone anchor 14 to bone. In the illustrated exemplary embodiment, for example, the distal shaft 18 includes an external thread 24. The external thread 24 may extend along at least a portion of the shaft 18. For example, in the illustrated exemplary embodiment, the external thread 24 extends from the distal tip 26 of the shaft 18 to proximate the head 16 of the bone anchor 14. One skilled in the art will appreciate that bone engagement mechanisms other than external thread 24 may be employed, including, for example, one or more annular ridges, multiple threads, dual lead threads, variable pitched threads, and/or any other conventional bone engagement mechanism. In the illustrated exemplary embodiment, the shaft diameter 20 of shaft 18 may be defined by the major diameter of external thread 24.

The proximal head 16 of the exemplary bone anchor 14 may be configured to facilitate adjustment of the bone anchor 14 relative to the receiving member 40 of the bone anchor assembly 10, as described below. For example, the head 16 may be generally spherical in shape to permit pivoting of the bone anchor 14 relative to the receiving member 40. In illustrated exemplary embodiment, for example, the head 16 may be in the shape of a truncated sphere having a generally planar proximal surface 30 and a generally hemispherically shaped distal surface 32. The head 16 of the bone anchor may have surface texturing, knurling, and/or ridges. The head 16 may also consist of one or more spherical sections of different diameter. The center of each section may or may not reside on the same point.

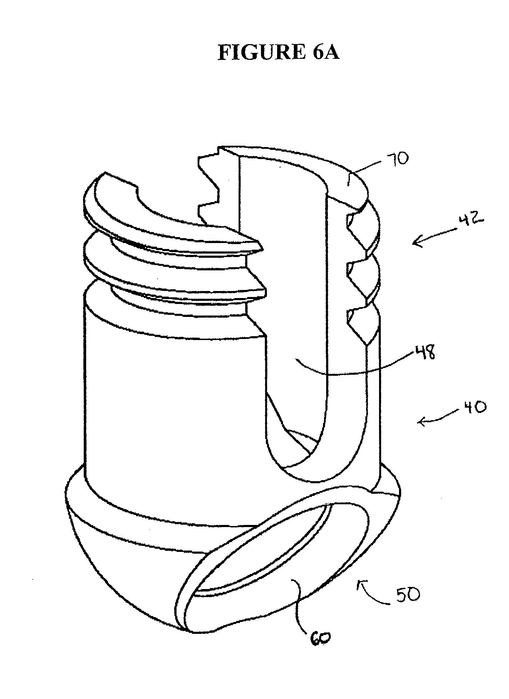

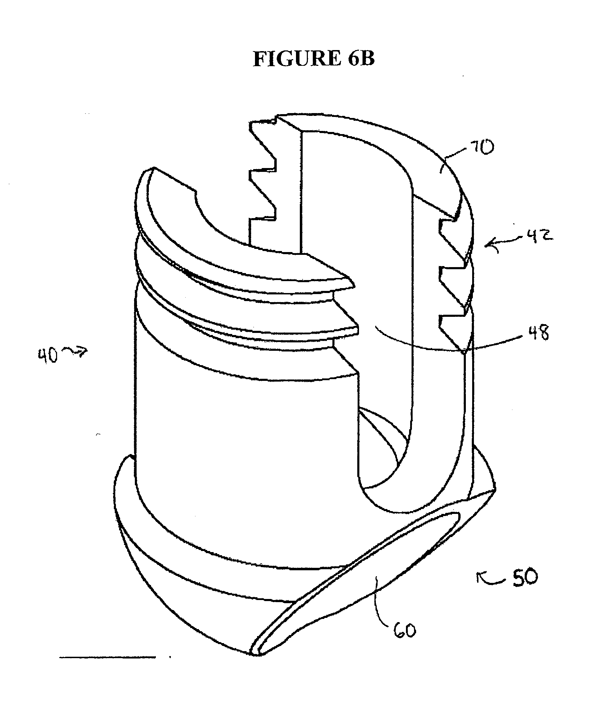

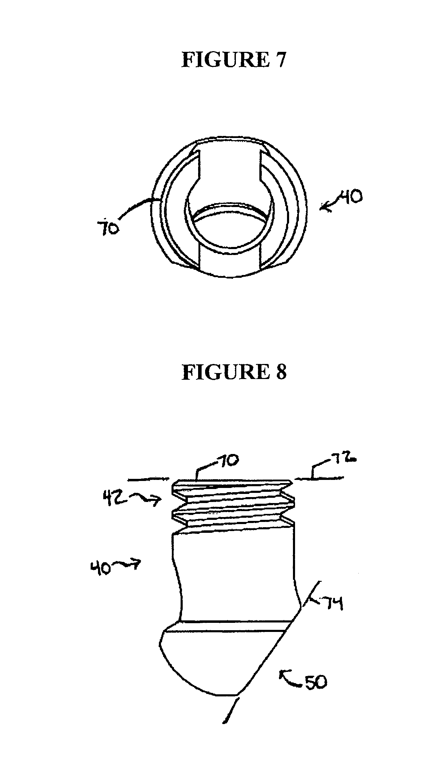

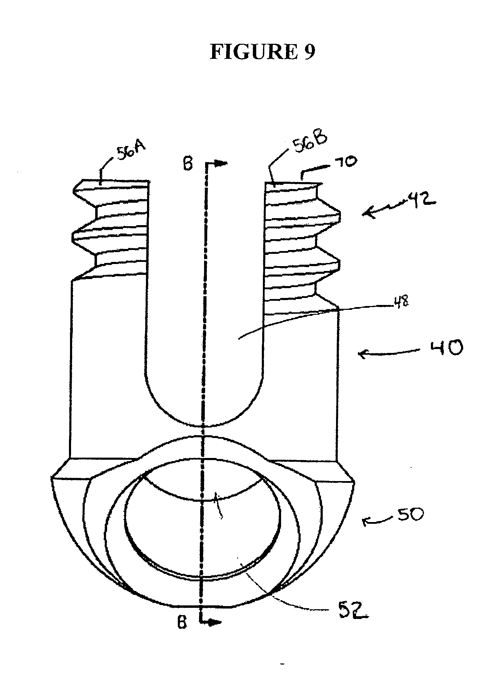

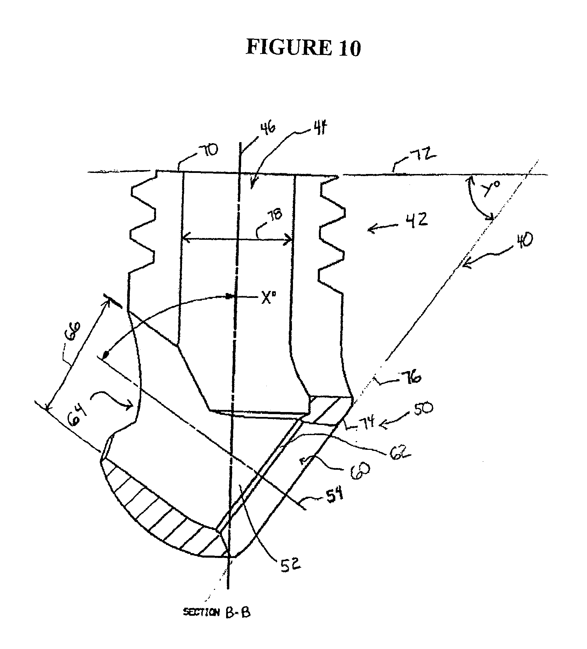

Referring to FIGURES 6-10, the receiving member 40 of the exemplary bone anchor assembly 10 includes a proximal first end 42 having a first bore 44 defining a first bore axis 46, a recess 48 in communication with the first bore 44, and a distal second end 50 having a second bore 52. In the exemplary embodiment, the second bore 52 defines a second bore axis 54 that intersects the first bore axis 46, as discussed in more detail below.

The receiving member 40, in certain exemplary embodiments, may be configured to receive a spinal fixation element and couple the spinal fixation element to the bone anchor assembly. In the exemplary embodiment, for example, the recess 48 of the receiving member 40 may be sized and shaped to receive a spinal rod 12, as illustrated in FIGURES 1-3. For example, the receiving member 40 has a generally U-shaped cross-section defined by two legs 56A and 56B separated by recess 48. Each leg 56A, 56B is free at the first end 42 of the receiving member 40. The exemplary spinal rod 12 may be seated within the recess 48 by aligning the spinal rod 12 and the recess 48, advancing the spinal rod 12 through the first bore 44 into the recess 48. The configuration of recess 48 of the receiving member 40 may be varied to accommodate the type, size and shape of spinal fixation element employed. In alternative exemplary embodiments, the exemplary spinal rod 14, or other spinal fixation element, may be coupled to the bone anchor assembly by alternative coupling mechanisms, in place of recess 48, including, for example, by an offset coupling mechanism, such as a band clamp, a sacral extender, or a lateral off-set connector.

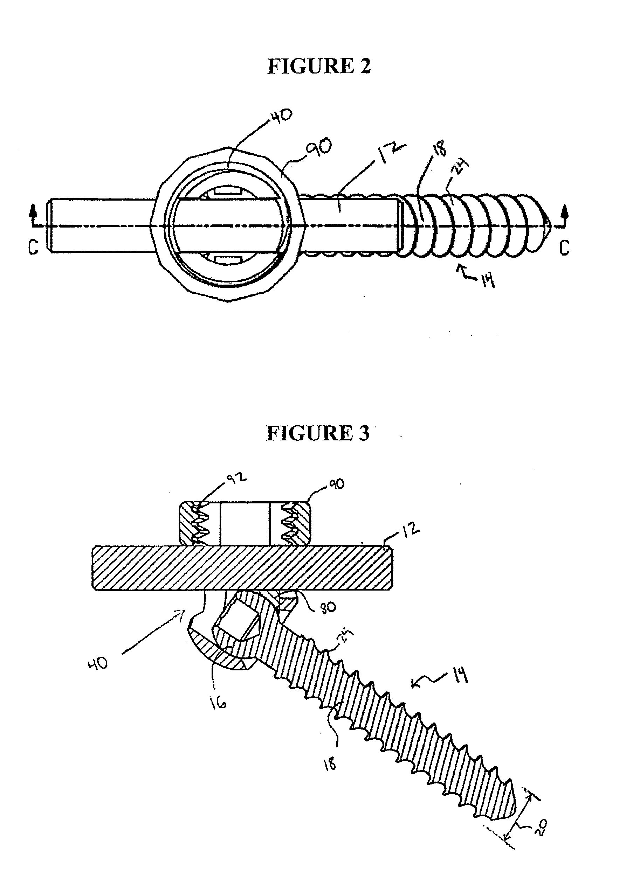

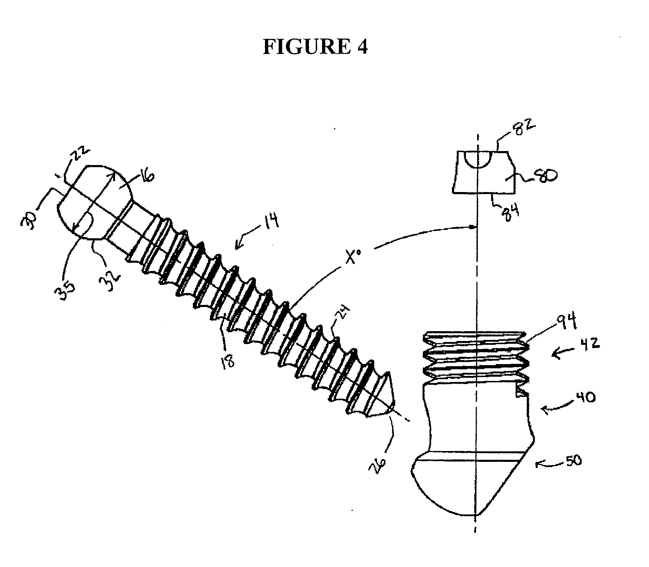

The receiving member 40 may couple a spinal fixation element to a bone anchor. In the exemplary embodiment, the second bore 52 may has a first opening 60 through which at least a portion of a bone anchor, such as exemplary bone anchor 14 described above, may extend. For example, the shaft 18 of the exemplary bone anchor 14 may extend through the first opening 60, as illustrated in FIGURES 3 and 4. The first opening 60 may be sized and shaped to engage the head 16 of the exemplary bone anchor 14. For example, the first opening 60 may define a seat 62 for engaging the head 16 of the exemplary bone anchor 14 that allows the bone anchor 14 to pivot relative to the receiving member 40. In some exemplary embodiments, the seat 62 may be generally spherical in shape to permit pivoting of the bone anchor 14 relative to the receiving member. In the illustrated exemplary embodiment, the seat 62 may be generally hemispherical in shape and may have a curvature analogous to the distal surface 32 of the head 16 of the exemplary bone anchor 14. In other exemplary embodiments, the seat 62 may be tapered or may have any other shape that allows adjustment of the head of the bone anchor relative to the receiving member. In the exemplary embodiment, the bone anchor assembly 10 is a polyaxial bone anchor assembly as the bone anchor 14 may be pivoted to one or more angles relative to the receiving member 40. In particular, the bone anchor 14 may be adjusted such that the longitudinal axis 22 of the bone anchor 14 is at angle of 0° to 90° relative to the second bore axis 54. In other exemplary embodiments, the seat 62 may be provided by a separate component that fits within the receiving member, such as a snap ring.

One skilled in the art will appreciate the bone anchor assemblies disclosed herein are not limited to the exemplary bone screw 14. In alternative exemplary embodiments, other bone anchors may be employed, including, for example, a monoaxial bone screw in which the bone screw is fixed relative to the receiving member, or a polyaxial or monoaxial hook or bolt.

In the exemplary embodiment, the second bore 54 of the receiving member 40 may have a second opening 64 opposite the first opening 60. The second opening 64 may be sized to facilitate connection of a bone anchor to the receiving member and/or to facilitate delivery of an instrument to the bone anchor once the bone anchor is coupled to the receiving member 40. For example, the second opening 64 may be sized to pass the head of a bone anchor during assembly of the bone anchor assembly. In the exemplary embodiment, the second opening 64 may have an extent 66, e.g., a diameter, that is greater than the diameter 35 of the head 16 of the exemplary bone anchor 14. In some exemplary embodiments, the second opening 64 may have an extent 66 that is less than or equal to the diameter 35 of the head 16 of the exemplary bone anchor 14. In such embodiments, the bone anchor may be assembled to the receiving member 40 by inserting the head of the bone anchor through the first opening 60 and a retaining member, such as, for example, a snap ring may be employed to provide the seat 62. The second opening 64 may have an extent 66, e.g., a diameter, that is greater than the extent of one or more instruments selected to engage the bone anchor.

The second bore axis 54 may be oriented at an angle to the first bore axis 46 to provided a preferred angle of orientation to the bone anchor. For example, the second bore axis 54 can be oriented at an angle X of approximately 0° to approximately 90° relative to the first bore axis 46. In bone anchor assemblies designed for use in the cervical region of the spine, the second bore axis 54 may be oriented at an angle X of approximately 40° to approximately 70° relative to the first bore axis 46, and, in a preferred embodiment, the second bore axis 54 may be oriented at an angle X of approximately 55° relative to the first bore axis 46.

In the illustrated exemplary embodiment, the first end 42 has a proximal surface 70 that defines a first plane 72 and the second end 50 has a distal surface 74 that defines a second plane 76. The first plane 72 may intersect the second plane 76 in the exemplary embodiment such that the second plane 76 is oriented at angle Y relative to the first plane 72. In the exemplary embodiment, the angle Y may be approximately equal to the angle X. In other exemplary embodiments, the angle Y may be distinct from the angle X.

As discussed above, the second opening 64 may be employed to facilitate coupling of the bone anchor to the receiving member 40 and/or to facilitate delivery of a tool to the bone anchor after assembly of the bone anchor and receiving member. In certain exemplary embodiments, the first bore 44 may have an extent 78, e.g., a diameter, that is less than the diameter 35 of the head 16 of the exemplary bone anchor 14. The extent 78 of the first bore 44 may be less than the shaft diameter 20 of the bone anchor 14. For example, in the case of the exemplary bone anchor 14, the extent 78 of the first bore 44 may be less than the major diameter of the threads 24 provided on the shaft 18 of the bone anchor 14. One skilled in the art will appreciate that the extent 78 of the first bore 44 may be greater than, equal to, or less than the extent of any or all the portions of the selected bone anchor.

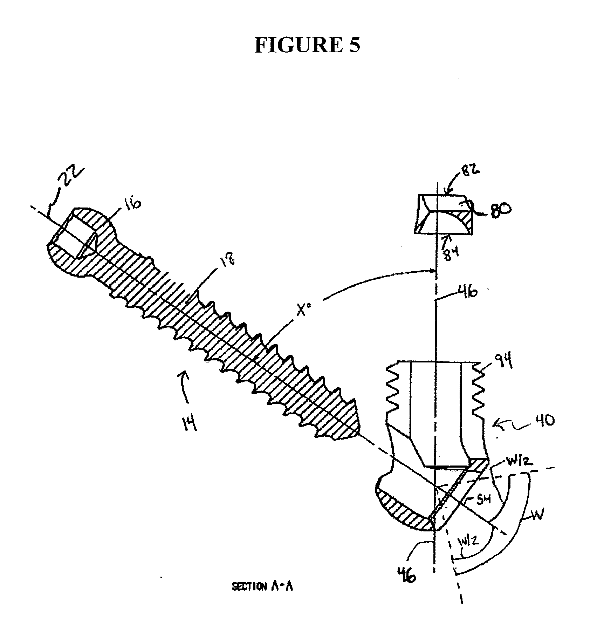

By providing a second opening 64 through which the bone anchor may be assembled to the receiving member, the value of angle X between the first bore axis 46 and the second bore axis 54 may be increased compared with conventional bone anchor assemblies lacking the second opening 64. Referring to FIGURE 5, for example, the bone anchor 14 may be symmetrically adjusted by angle W about a neutral orientation in which the longitudinal axis 22 of the bone anchor 14 is coaxial to the second bore axis 54. For example, the bone anchor 14 may be adjusted by an angle W/2 in the direction of the first bore axis 46 and may be adjusted by an angle W/2 away from the first bore axis 46. In the illustrated exemplary embodiment, the angle X may be greater than or equal to the angle W/2.

The bone anchor assembly 10 may optionally include a compression member 80 positionable within the receiving member 40 between the spinal fixation element and the bone anchor. As illustrated in FIGURES 2-3, the compression member 80 may be positioned within the first bore 44 and the recess 48 between the spinal rod 12 and the head 16 of the exemplary bone anchor 14. In the exemplary embodiment, the compression member 80 may have a proximal first surface 82 for engaging the spinal fixation element and an opposing distal second surface 84 for engaging the head 16 of the bone anchor 14.

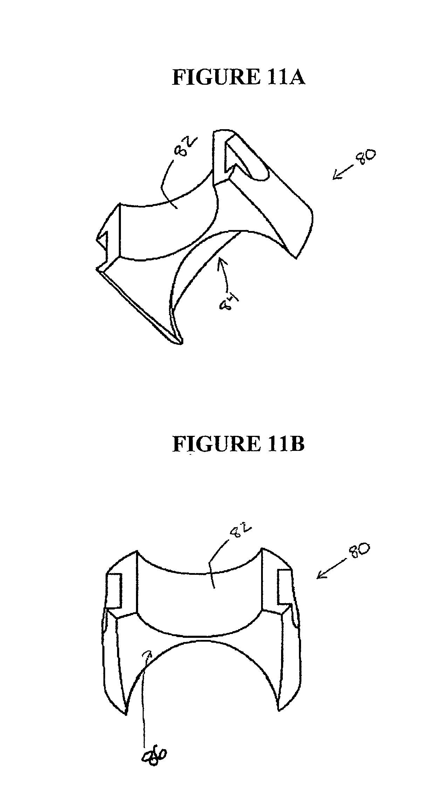

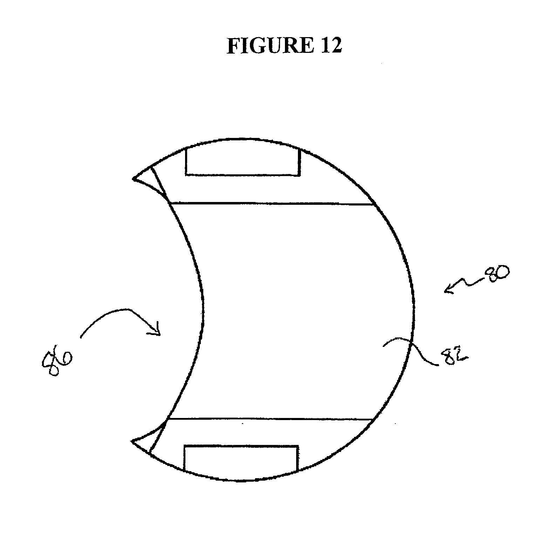

Referring to FIGURES 11A, 11B, and 12, the exemplary embodiment of the compression member 80 may be generally disc-shaped having a circular cross-section or other cross section preferably analogous to the cross-section of the first bore 44 of the receiving member 40. The first surface 82 of the compression member 80 may be configured to seat the spinal fixation element. In the exemplary embodiment, the first surface 82 has a generally arcuate cross-section having a curvature that may approximate the curvature of the exemplary spinal rod 14. The second surface 84 may be configured to engage the head of the bone anchor. For example, the second surface 84 may have a generally spherical shape or a tapered shape to engage the head of the bone anchor. In the exemplary embodiment, the second surface 84 may have be hemispherical in shape and may have a curvature approximating the curvature of the head 16 of the bone anchor 14. The compression member 80 may have a cut-out 86 that facilitates positioning of an instrument or component of the bone anchor through the second bore 52. The cut-out may be generally arcuate in shape and may extend between the first and second surfaces 82, 84 of the exemplary compression member 80.

The exemplary bone anchor assembly 10 may include a closure mechanism 90 that secures the spinal fixation element to the bone anchor assembly. Referring to FIGURES 1-3, the closure mechanism 90 secures the exemplary spinal rod 12 within the recess 48 of the receiving member 40. The closure mechanism 90 may engage the first end 42 of the receiving member 40 or, in other exemplary embodiments, may engage other portion(s) of the receiving member 40. The exemplary closure mechanism 90 is an external cap that engages an outer surface of the first end 42 of the receiving member 40. For example, the closure mechanism 90 may have internal threads 92 that engage external threads 94 provided on the first end 42 of the receiving member 40. Distal advancement of the closure mechanism 90 into engagement of the spinal rod 12, secures the spinal rod 12 within the recess 48 of the receiving member 40. In embodiments employing a compression member 80, such as exemplary bone anchor 10, distal advancement of the closure mechanism 90 into engagement with the spinal rod 12 seats the spinal rod 12 in the compression member 80. Distal advancement of the spinal rod 12 may also fix the bone anchor 14 relative to the receiving member 40 by engagement of the spinal rod 12 against the head 16 of the bone anchor 14 or by engagement of the compression member 80 against the head 16 of the bone anchor, as in the case of the illustrated exemplary embodiment.

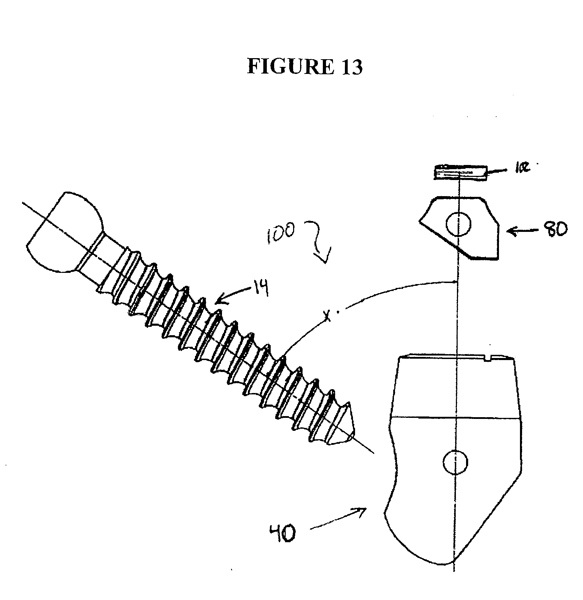

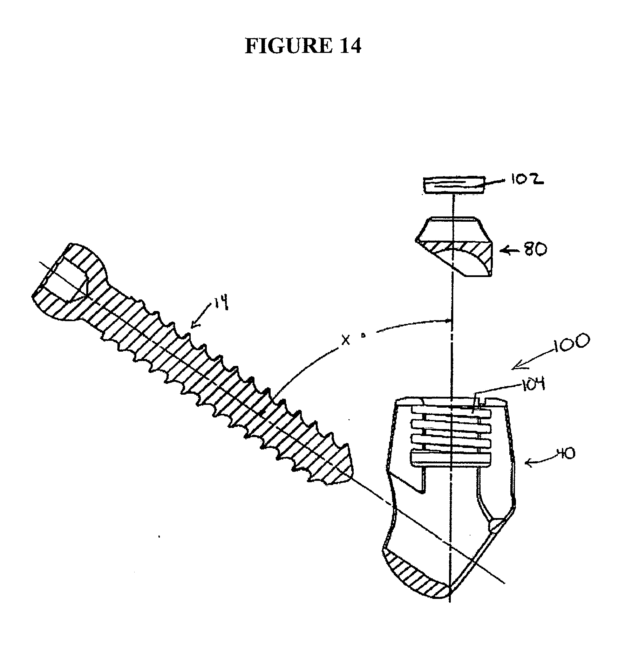

One skilled in the art will appreciate that other types of closure mechanisms may be employed. For example, an internal closure mechanism positionable within the first bore 44 of the receiving member 40 may be employed. For example, FIGURES 13 and 14, illustrate an exemplary embodiment of a bone anchor assembly 100 having internal threads 104 for engagement by an internal closure mechanism 102 having external threads. In other exemplary embodiments, the closure mechanism may comprise an external and an internal closure mechanism, a non-threaded twist-in cap, and/or any other conventional closure mechanism.

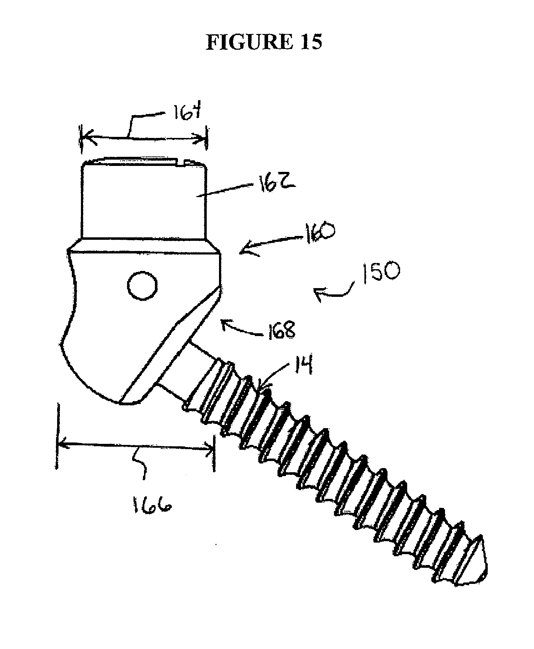

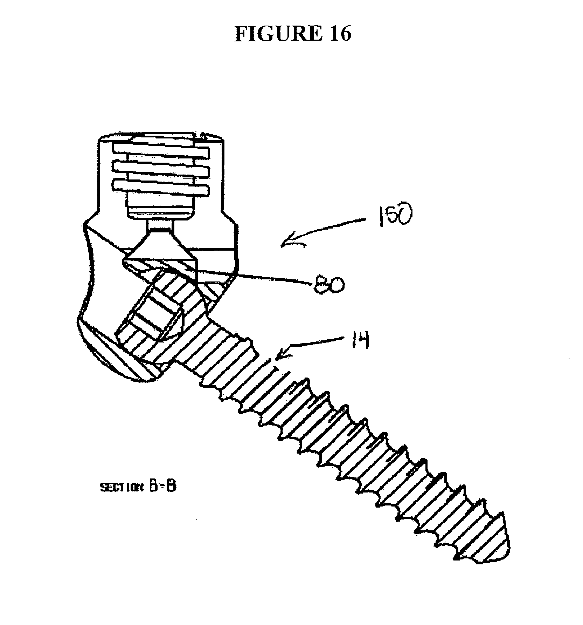

FIGURES 15 and 16 illustrate an exemplary embodiment of a bone anchor assembly 150 in which the receiving member 160 has a proximal first end 162 having an extent 164, e.g., a diameter, that is less than the extent 166 of the distal second end 168 of the receiving member 160. Reduction of the extent 164 of the first end 162 can minimize interference between bone anchor assemblies positioned on adjacent vertebrae or otherwise implanted in proximity to one another.

The components of the bone anchor assembly may be manufactured from any biocompatible material, including, for example, metals and metal alloys such as titanium and stainless steel, polymers, and/or ceramics. The components may be manufactured of the same or different materials. In one exemplary method of manufacturing, the bone anchor and receiving member are separately constructed and assembled prior to implantation. The bone anchor, in one exemplary method, may be coupled to the receiving member by positioning the bone anchor through the second opening 64 in the second bore 52. The head of the bone anchor may be seated against seat 62 of the first opening 60 such that the shaft 18 of the bone anchor 14 extends through the first opening 60. The compression member 80 may be positioned through the first bore 44 into engagement with the head of the bone anchor before, or after, implantation of the bone anchor assembly.

The bone anchor assembly 10 may be implanted by any conventional procedure. In one exemplary method of engaging the bone anchor assembly to a vertebra of the spine, the bone anchor assembly may be delivered to proximate the vertebra through an open incision or, in a minimally invasive procedure, though a percutaneous pathway between a minimally invasive skin incision and the vertebra. A tool, such as a bone anchor driver, may be inserted through the second opening 64 in the second bore 52. The tool may engage the head of the bone anchor and may be employed to secure the bone anchor to the vertebra by, for example, rotating the proximal end of the tool. The tool can drive the bone anchor into a pre-drilled hole in the vertebra or, in the case of self-drilling bone screws for example, the tool can rotate the bone anchor and create a hole in bone as the bone anchor is advanced. Depending on the procedure, a spinal fixation element may be coupled to the bone anchor assembly. The spinal fixation element may be coupled to the bone anchor assembly before, during, or after the bone anchor assembly engages the bone. A closure mechanism may be used to secure the fixation element to the bone anchor assembly.

In either an open or minimally invasive procedure the action of driving the bone anchor by positioning the bone anchor driver through second opening 64 may occur through an incision or percutaneous opening that is distinct from the incision or percutaneous opening through which the spinal fixation element or closure mechanism is inserted. For example, the bone anchor assembly may be delivered proximate to the spine through one incision or percutaneous opening, and the bone anchor driver may be delivered through a second incision or percutaneous opening to engage the bone anchor through second opening 64.

In one exemplary method, the bone anchor of the bone anchor assembly may engage two or more adjacent vertebrae. For example, in C1-C2 transarticular fixation, the shaft of the bone anchor may be inserted through the facet joint of the C1 vertebra and the C2 vertebra. Such a procedure eliminates the need for a bone anchor assembly for each vertebra.

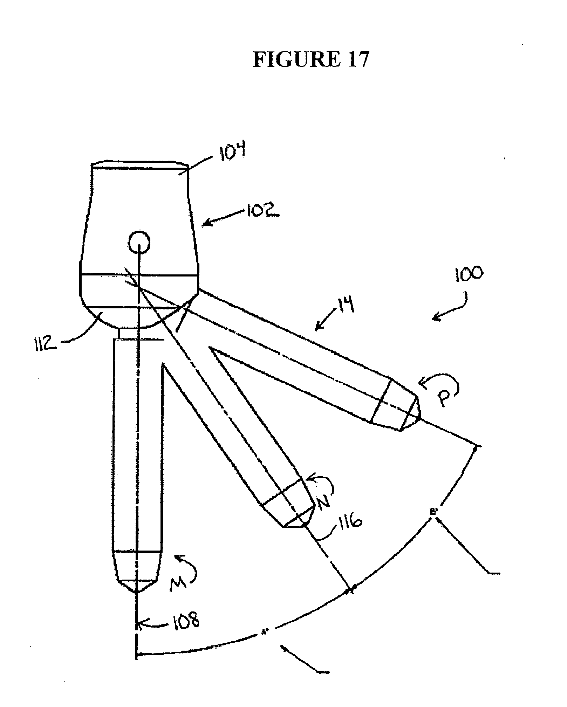

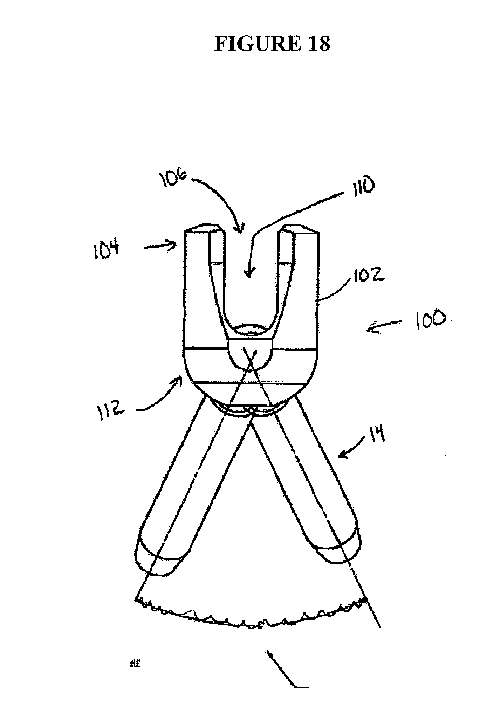

FIGURES 17-21 illustrate an exemplary embodiment of a bone anchor assembly 100 having a receiving member 102 having a first end 104 having a first bore 106 defining a first bore axis 108, a recess 110 in communication with the first bore 106, and a second end 112 having a second bore 114 sized to receive at least a portion of a bone anchor 14. As in the case of the exemplary bone anchor assembly 10 described above, the recess 110 may be sized and shaped to receive a spinal fixation element, such as, for example, a spinal rod. In the exemplary embodiment, the second bore 114 may define a second bore axis 116 that may intersect the first bore axis 108 at an angle X. The second bore 114, in the exemplary embodiment, may have a first opening 118 through which the at least a portion of the bone anchor 14 may extend.

In the illustrated exemplary embodiment, the first bore 106 has a proximal opening 120 defining a first plane 122 and a portion of the first opening 118, which in the exemplary embodiment is distal to the proximal opening 120 of the first bore 106, defines a second plane 124. The first plane 122 may intersect the second plane 124 in the exemplary embodiment such that the second plane 124 is oriented at the angle Y relative to the first plane 122. In the exemplary embodiment, the angle Y may be approximately equal to the angle X. In other exemplary embodiments, the angle Y may be distinct from the angle X.

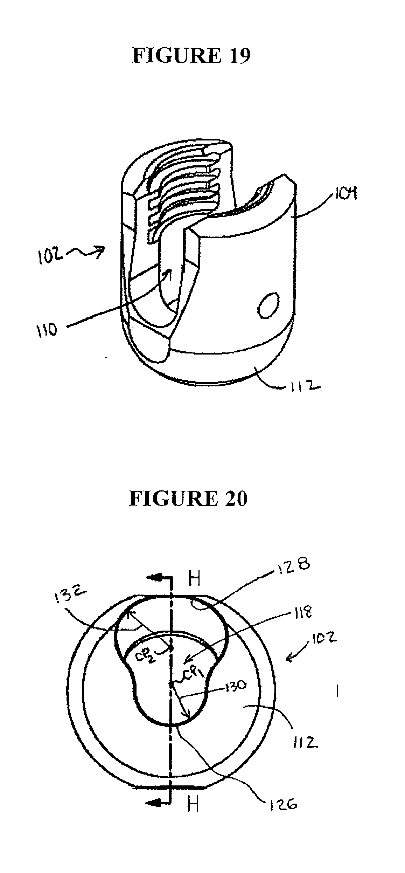

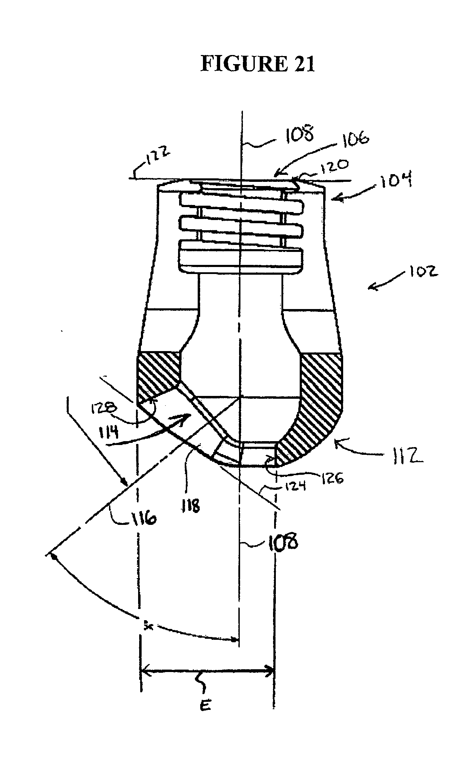

In the exemplary embodiment, the first opening 118 is configured to allow a portion of a bone anchor, such as the shaft 18 of the exemplary bone anchor 14, to be inserted therethrough during assembly of the bone anchor assembly 100. For example, the first opening 118 may be generally oblong in shape, as in the illustrated exemplary embodiment, and may be intersected by the first bore axis 108 and the second bore axis 116, as illustrated in FIGURES 20 and 21. In the exemplary embodiment, the first opening 118 may have a first arcuate end 126 spaced apart a distance E from a second arcuate end 128. The distance E between the first arcuate end 126 and the second arcuate end 128 may be selected such that the first bore axis 108 and the second bore axis 116 intersect the first opening 118. The first arcuate end 126 may have a center CP₁ that is proximate the first bore axis 108 and the second arcuate end may have a center CP₂ that is proximate the second bore axis 116. In certain exemplary embodiments, such as the illustrated exemplary embodiment, the first arcuate end 126 may have a center CP₁ that is intersected by the first bore axis 108 and the second arcuate end may have a center CP₂ that is intersected by the second bore axis 116.

The first arcuate end 126 may have a first radius of curvature 130 distinct from the second radius of curvature 132 of the second arcuate end 128. For example, the first radius of curvature 130 may be less than the second radius of curvature 132, as in the case of the illustrated exemplary embodiment. The first radius of curvature 130 may be greater than the shaft diameter of the bone anchor to facilitate insertion of the bone anchor to the receiving member 102 during assembly. The first bore 106 may include internal threads proximate the first opening 118 for engagement with threads provided on the shaft of the bone anchor to facilitate passage of the shaft through the first opening 118. The threads may extend to the first arcuate end 126, allowing the first end 126 to have a radius of curvature less than the shaft diameter of the bone anchor.

In other exemplary embodiments, the first arcuate end 126 may have a radius of curvature 130 approximately equal to the radius of curvature 132 of the second arcuate end 128. In such embodiments, the first opening 118 may be generally elliptical in shape.

In one exemplary method of manufacturing, a bone anchor, such as exemplary bone anchor 14, may be inserted into the receiving member 102 through the first bore 106. During insertion, the longitudinal axis of the bone anchor may be aligned with the first bore axis 108. At least a portion of the bone anchor, e.g., the shaft of the bone anchor, may be advanced through the first opening 118 of the second bore 114. During advancement, the longitudinal axis of the bone anchor may remain aligned with the first bore axis 108. The head of the bone anchor may then be seated against the seat provided by the first opening 118.

In polyaxial embodiments, as in the illustrated exemplary embodiment, the bone anchor 14 may be adjustable relative to the receiving member 102. For example, the bone anchor 14 may be adjusted from a neutral position, in which the longitudinal axis of the bone anchor 14 is coaxial with the second bore axis 116, as indicated by arrow N in FIGURE 17. The size and shape of the first opening 118 can define the extent of adjustment of the bone anchor. For example, the bone anchor 14 may be adjusted toward the first arcuate end 126 by an angle A' to an offset position in which the longitudinal axis of the bone anchor 14 is coaxial with the first bore axis 108, as indicated by the arrow M in FIGURE 17. The bone anchor 14 may be adjusted toward the second arcuate end 128 by an angle B', as indicated by the arrow P in FIGURE 17. In certain exemplary embodiments, such as the bone anchor assembly 10 described above, the angle A' and the angle B' may be approximately equal. In other exemplary embodiments, such the bone anchor assembly 100, the angle A' and the angle B' may be distinct from one another, in which case the bone anchor is asymmetrically adjustable about the second bore axis. For example, A' may be greater than B', as in the case of bone anchor assembly 100.

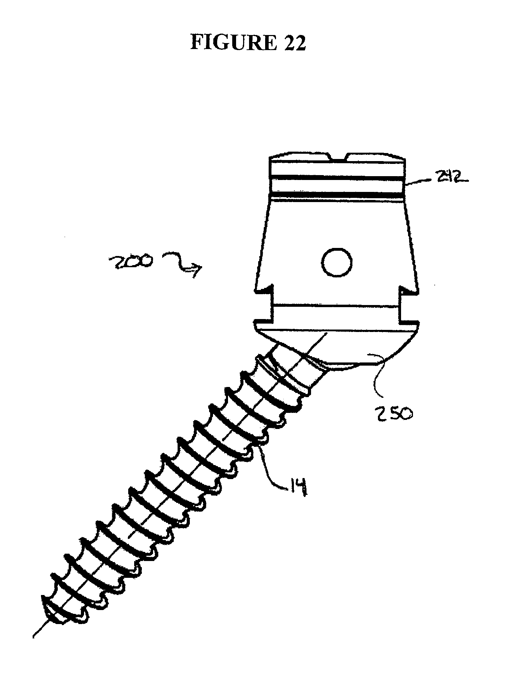

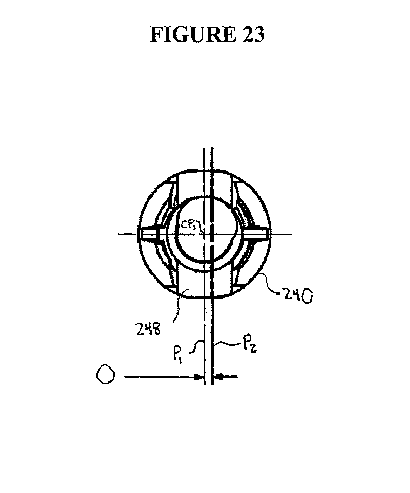

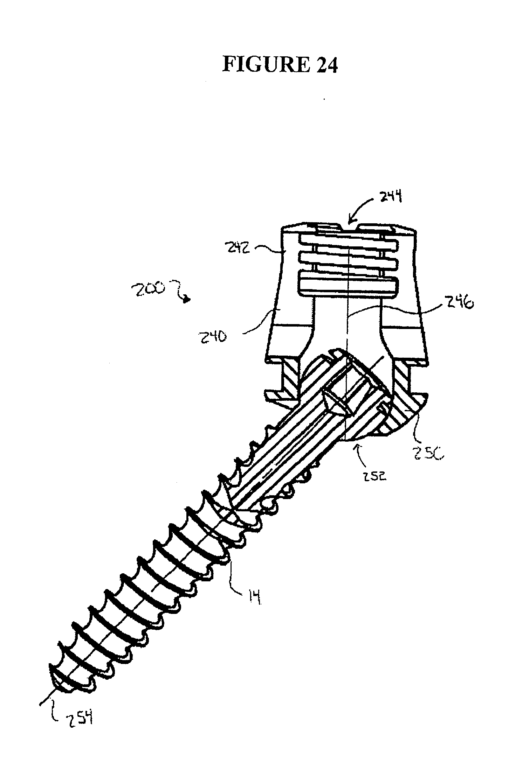

Another exemplary embodiment of a bone anchor assembly 200 is illustrated in FIGURES 22-24. The receiving member 240 of the exemplary bone anchor assembly 200 includes a proximal first end 242 having a first bore 244 defining a first bore axis 246, a recess 248 in communication with the first bore 244, and a distal second end 250 having a second bore 252. In the exemplary embodiment, the second bore 252 defines a second bore axis 254 that is offset a distance O from the first bore axis 246. As a result of the offset O, the first bore axis 246 and the second bore axis 254 lie in separate planes and do not intersect each other. Referring to FIGURE 23, for example, the first bore axis 246 passes through an approximate center point CP₁ of the first bore 244 and lies in a first plane P₁. The second bore axis 254 passes through an approximate center point of the second bore 252 and lies in a second plane P₂, which is offset from the first plane P₁ by an offset distance O. In the illustrated exemplary embodiment, the second bore 252 may be conical. In other exemplary embodiments, the second bore may be cylindrical or of any other suitable shape. In the illustrated embodiment, the first plane P₁ and second plane P₂ are both parallel to the axis of recess 248. One skilled in the art will appreciate that the first plane P₁ and second plane P₂ may be oriented at any angle from 0° to 180° relative to the axis of recess 248.

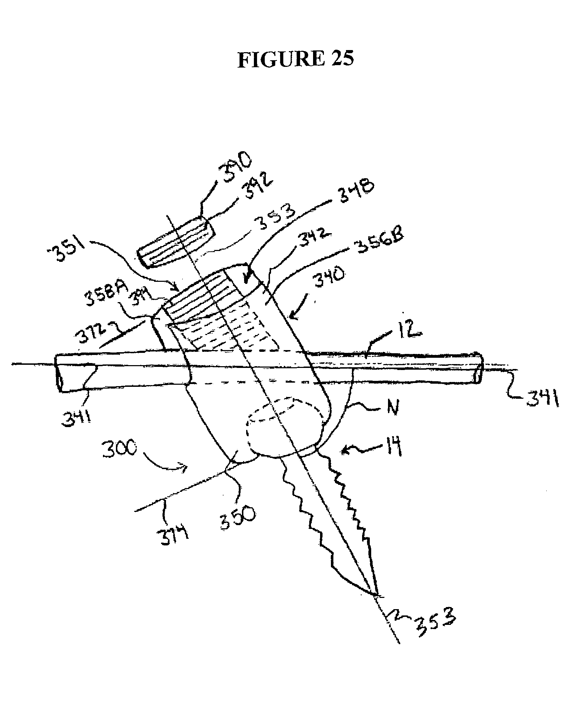

FIGURE 25 illustrates a further exemplary bone anchor assembly 300 having a receiving member 340 including a proximal first end 342, a distal second end 350, and a bore 351 extending therebetween. In the exemplary embodiment, the receiving member 340 includes a recess 348 sized and shaped to receive a fixation element, for example, a spinal rod 12. For example, the receiving member 340 may have a generally U-shaped cross section defined by legs 356A and 356B separated by recess 348. In the exemplary embodiment, the axis 341 of the recess 348 is oriented at an angle N of approximately 0° to approximately 90° relative to the axis 353 of the bore 351 of the receiving member 340. In bone anchor assemblies designed for use in the cervical region of the spine, the recess axis 341 may be oriented at an angle N of approximately 15° to approximately 70° relative to the bore axis 353, and, in preferred embodiments, the recess axis 341 may be oriented at an angles N of approximately 55° and 15° relative to the bore axis 353.

The proximal end 342 of the receiving member 340 may include internal threads 394 for receiving external threads 392 provided on a closure mechanism 390, e.g., a set screw. In the exemplary embodiment, the axis of the internal threads 394 of the receiving member 340 is oriented approximately parallel to the bore axis 353. In such an exemplary embodiment, the closure mechanism 390 is advanced in a direction parallel to the bore axis 353 into contact with the rod 12. In addition, in such an exemplary embodiment, the closure mechanism 390 is advanced at angle parallel to the bore axis 353 and at an angle other than perpendicular to the longitudinal axis of the rod 12.

In the exemplary embodiment, the first end 342 of the receiving member 340 defines a first plane 372 and the second end 350 defines a second plane 374 that is oriented approximately parallel to the first plane 372. The recess axis, in the exemplary embodiment, intersects the first plane 372 and the second plane 374. The axis of the internal threads 394 are approximately perpendicular to the distal second plane 374, which may allow the bone anchor driver to engage the internal threads and rigidly lock to the bone anchor assembly 300, thereby facilitating insertion of the bone anchor assembly. When advancing the bone anchor assembly 300 into the bone, the perpendicular nature of the second plane 374 to the axis of rotation allows the bone anchor assembly 300 to be inserted with minimal interference with the anatomy.

In another aspect, the invention provides a bone anchor assembly comprising:
a bone anchor having a proximal head and a distal shaft configured to engage bone, the distal shaft having a shaft diameter and a longitudinal axis, and
a receiving member for receiving a spinal fixation element to be coupled to the bone anchor, the receiving member having
a first end having a first bore defining a first bore axis and having a proximal opening defining a first plane,
a recess in communication with the first bore, the recess being sized and shaped to receive a spinal fixation element,
a second end having a second bore sized to receive at least a portion of the bone anchor, the second bore defining a second bore axis, the second bore having a first opening through which the at least a portion of the bone anchor extends and a second opening opposite the first opening, the first opening defining a second plane that intersects the first plane, the second opening being sized to pass the head of the bone anchor during assembly of the bone anchor assembly.

In a further aspect, the invention provides bone anchor assembly comprising:
a bone anchor having a proximal head and a distal shaft configured to engage bone, the distal shaft having a shaft diameter and a longitudinal axis, and
a receiving member for receiving a spinal fixation element to be coupled to the bone anchor, the receiving member having
a first end having a first bore defining a first bore axis and having a proximal opening defining a first plane,
a recess in communication with the first bore, the recess being sized and shaped to receive a spinal fixation element,
a second end having a second bore sized to receive at least a portion of the bone anchor, the second bore defining a second bore axis, the second bore having a first opening defining a second plane and through which the at least a portion of the bone anchor extends, the second plane intersecting the first plane, the first opening being generally oblong in shape and being intersected by the first bore axis and the second bore axis.

The invention also provides a bone anchor assembly comprising:
a bone anchor having a proximal head and a distal shaft configured to engage bone, the distal shaft having a shaft diameter and a longitudinal axis, and
a receiving member for receiving a spinal fixation element to be coupled to the bone anchor, the receiving member having
a first end, a second end, and a bore defining a bore axis extending between the first end and the second end, and
a recess in communication with the first bore, the recess defining a recess axis and being sized and shaped to receive a spinal fixation element, the recess axis being oriented at angle other than perpendicular relative to the bore axis.

The invention also provides a bone anchor assembly comprising: a bone anchor having a proximal head and a distal shaft configured to engage bone, the distal shaft having a shaft diameter and a longitudinal axis; and a receiving member for receiving a spinal fixation element to be coupled to the bone anchor, the receiving member having a first end having a first bore defining a first bore axis, a recess in communication with the first bore, the recess being sized and shaped to receive a spinal fixation element, a second end having a second bore sized to receive at least a portion of the bone anchor, the second bore defining a second bore axis that intersects the first bore axis, the second bore having a first opening through which the at least a portion of the bone anchor extends, the first opening being generally oblong in shape and being intersected by the first bore axis and the second bore axis.

The first opening may have a first arcuate end spaced apart a distance from a second arcuate end. The first arcuate end may have a radius of curvature distinct from a radius of curvature of the second end. The first arcuate end may have a center that is proximate the first bore axis and the second arcuate end may have a center that is proximate the second bore axis. The first arcuate end may have a radius of curvature that is greater than the shaft diameter. The first opening may be approximately elliptical in shape. The bone anchor may be asymmetrically adjustable about the second bore axis.

## Claims

1. A bone anchor assembly comprising:
a bone anchor having a proximal head and a distal shaft configured to engage bone, the distal shaft having a shaft diameter and a longitudinal axis, and
a receiving member for receiving a spinal fixation element to be coupled to the bone anchor, the receiving member having
a first end having a first bore defining a first bore axis,
a recess in communication with the first bore, the recess being sized and shaped to receive a spinal fixation element,
a second end having a second bore sized to receive at least a portion of the bone anchor, the second bore defining a second bore axis that intersects the first bore axis, the second bore having a first opening through which the at least a portion of the bone anchor extends and a second opening opposite the first opening, the second opening being sized to pass the head of the bone anchor during assembly of the bone anchor assembly,
in which the first end of the receiving member has an outer diameter that is less than an outer diameter of the second end of the receiving member.

2. The bone anchor assembly of claim 1, which includes a compression member which can be positioned within the receiving member between the fixation element and the head of the bone anchor, the compression member having a first surface for engaging the spinal fixation element and an opposing second surface for engaging the head of the bone anchor.

3. The bone anchor assembly of claim 2, in which the compression member includes an arcuate cut-out to facilitate positioning of the bone anchor through the second opening.

4. The bone anchor assembly of claim 1, in which the shaft of the bone anchor includes external threads along at least a portion of the length thereof, the external threads having a major diameter, in which the diameter of the first bore is less than the major diameter of the external threads.

5. The bone anchor assembly of claim 1, in which the second bore axis is oriented at angle of approximately 40° to approximately 70° relative to the first bore axis.

6. The bone anchor assembly of claim 1, in which the spinal fixation element is a spinal rod.

7. The bone anchor assembly of claim 1, in which the head of the bone anchor includes a generally hemispherically shaped distal surface that engages a generally hemispherically shaped seat provided at the first opening of the second bore.

8. The bone anchor assembly of claim 1, in which the bone anchor is adjustable relative to the receiving member.

9. The bone anchor assembly of claim 1, further comprising a closure mechanism engageable to the first end of the receiving member, the closure mechanism securing the spinal fixation element within the recess when engaged to the first end of the receiving member.

10. The bone anchor assembly of claim 9, in which the closure mechanism engages an outer surface of the first end of the receiving member.

11. The bone anchor assembly of claim 9, in which the closure mechanism seats within the first bore of the receiving member.

12. The bone anchor assembly of claim 1, in which the first bore has a diameter less than the shaft diameter.

13. The bone anchor assembly of claim 1, in which the first end defines a first plane, the second end defines a second plane, and the first and second plane intersect one another.
